(19) Europäisches Patentamt
European Patent Office
Office européen des brevets

(11) Publication number: 0 236 591 B1

(12) EUROPEAN PATENT SPECIFICATION

(45) Date of publication of patent specification: 22.11.90

(51) Int. Cl.$^5$: C 07 C 231/06

(21) Application number: 86202258.9

(22) Date of filing: 15.12.86

# (54) Process for the preparation of alpha-hydroxycarboxylic acid amides.

(30) Priority: 22.01.86 NL 8600130

(43) Date of publication of application:
16.09.87 Bulletin 87/38

(45) Publication of the grant of the patent:
22.11.90 Bulletin 90/47

(84) Designated Contracting States:
AT BE CH DE ES FR GB IT LI NL SE

(56) References cited:
FR-A-2 029 580

CHEMICAL ABSTRACTS, vol. 102, no. 13, 1st April 1985, page 671, abstract no. 113066u, Columbus, Ohio, US; & JP-A-59 161 341 (UBE INDUSTRIES, LTD) 12-09-1984

(73) Proprietor: STAMICARBON B.V.
Mijnweg 1
NL-6167 AC Geleen (NL)

(72) Inventor: Boesten, Wilhelmus Hubertus Joseph
Brountslaan 9
NL-6132 BJ Sittard (NL)
Inventor: Kamphuis, Johan
Aureliushof 129h
NL-6215 SR Maastricht (NL)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 236 591 B1

Courier Press, Leamington Spa, England.

**Description**

The invention relates to a process for the preparation of α-hydroxycarboxylic acid amides of the formula:

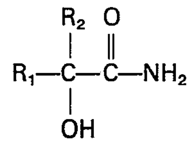

where $R_1$ represents alkyl, alkaryl, aryl, alkenyl, heteroalkyl or heteroaryl, whether or not substituted, $R_2$ represents hydrogen or methyl, or $R_1$ and $R_2$ together form cycloalkyl, by conversion of the corresponding cyanohydrins.

The α-hydroxycarboxylic acid amides obtained can be applied as intermediary in the preparation of optically active α-hydroxy acids. Both the amides and the acids can be applied in the pharmaceutical, the agricultural and the flavours and fragrances industries.

From Eliel, E. L. and Freeman, J. P., Organic Synthesis Coll. Vol. IV (1963), pp. 58—62, it is known to prepare α-methyl-α-hydroxycarboxylic acid amides by hydrolysis of the cyano group of a corresponding cyanohydrin using concentrated HCl during a reaction that takes 18 hours, after which the reaction mixture is cautiously neutralized. The drawback of such a process resides in the corrosive properties of concentrated HCl, the long reaction time, the formation of hydrocyanic acid in the mixture and the release of much inorganic salt during the neutralization reaction. Salt-free working is necessary notably if the amide obtained is converted into the L-acid and the D-amide using enzymatic methods, the reaction rate of an enzymatic conversion being adversely affected by high salt concentrations.

The object of the invention is elimination of the drawbacks referred to.

The invention therefore provides a process for the preparation of α-hydroxycarboxylic acid amides of the formula

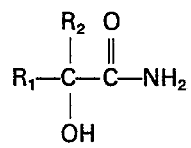

where $R_1$ represents alkyl, alkaryl, aryl, alkenyl, heteroalkyl or heteroaryl, whether or not substituted, $R_2$ represents hydrogen or methyl, or $R_1$ and $R_2$ together form cycloalkyl, by conversion of the corresponding cyanohydrins and is characterized in that the conversion takes place in the presence of hydrogen peroxide and in the presence of 0,5—3 moles ammonia per mole cyanohydrin.

Thus it is achieved that α-hydroxycarboxylic acid amides, α-hydroxy-α-cycloalkylcarboxylic acid amides can be prepared in a simple and economically sound manner.

Another advantage is that the α-hydroxycarboxylic acid amides obtained can simply be converted into the corresponding α-hydroxy acids using 1 to 2 equivalents of a strong base such as NaOH or KOH. It is true that an α-hydroxy acid can be prepared directly from the corresponding α-hydroxynitrile, but this is possible only if use is made of 6 equivalents of a strong acid such as HCl. The drawback then again is the highly corrosive environment.

It cannot but be called surprising that the nitrile group should be converted selectively in the process according to the invention, for from Tanaka, M. et al., Angew. Chem. (1984) *96,* pp. 519—520, it is known that in the presence of t-butyl hydroxyperoxide and a ruthenium catalyst various cyanohydrins are converted into the corresponding ketones, that is into 2-oxoalkane nitriles.

The process according to the invention is preferably carried out in the presence of 2—6 moles hydrogen peroxide, in particular in the presence of about 2.2 moles hydrogen peroxide, per mole cyanohydrin. It is generally attempted not to use more moles hydrogen peroxide per mole cyanohydrin than is strictly necessary.

The amount of ammonia to be applied in the process according to the invention may vary between 0.5 and 3 moles per $NH_3$ per mole cyanohydrin. In particular use is made of less than 2 moles $NH_3$. Use of more than 3 moles $NH_3$ may effect the conversion of hydroxy nitrile into amino nitrile; any conversion of hydroxy nitrile into amine nitrile, however, is dependent on the stability of the corresponding hydroxy nitrile. This stability determines the maximum allowable concentration of ammonia if a pure reaction product is desired. Upon termination of the reaction the excess hydrogen peroxide can be reduced using, for instance, palladium-on-carbon, after which the excess ammonia can be removed by, for instance, distillation. In this way the formation of salts, besides the desired α-hydroxycarboxylic acid amides, that subsequently need to be removed, is prevented. The basic environment present during the reaction also prevents the release of the extremely toxic hydrocyanic acid.

The process according to the invention can be carried out at various temperatures. By preference a

temperature between 0 and 50°C is applied, in particular between 25 and 40°C. As the cyanohydrins are thermally unstable, the reaction temperature is preferably kept low. The stability of the α-hydroxy nitrile determines the allowable temperature. The cyanohydrin decompose notably above 60°C into cyanide and ketones, upon which the cyanide may polymerize under the reaction conditions then prevailing.

The process is generally carried out in solvents such as water and lower alcohols, such as methanol, ethanol, propanol, isopropanol, butanol, etc. Higher alcohols can in principle also be used, but are less interesting because of the poorer compatibility with water. Furthermore other organic solvents can that can be mixed with water can be used, such as dioxane and tetrahydrofuran.

In this way the reaction is completed within a few hours, irrespective of whether $R_2$ is hydrogen or methyl, of whether $R_1$ and $R_2$ together form a cycloalkyl.

The reaction is not affected by $R_1$, either, which may therefore in principle be chosen at random. $R_1$ will generally be an alkyl, aryl, alkaryl, alkenyl, heteroalkyl or heteroaryl group, it of course being possible for the alkyl group to be cyclic. The bulkiness of this group is not important, either, but $R_1$ will mostly not contain more than 20 carbon atoms. Optionally $R_1$ may be a substituted hydrocarbon residue, insofar as the substituent does not react under the reaction conditions. As such, notably oxygen and nitrogen containing compounds can be mentioned, in particular alkoxy and alkyl amine. $R_2$ may also form a cycloalkyl together with $R_1$.

The optically active α-hydroxycarboxylic acid amides and corresponding acids will generally find application as intermediaries for the agricultural, the pharmaceutical and the flavours and fragrances industries. $R_1$ thus preferably is an organic group, whether or not substituted, that is closely related to corresponding groups of natural hydroxy acids. Examples are methyl, ethyl, isopropyl, 2-butyl, benzyl, methoxy-substituted benzyl groups, hydroxymethyl and methoxymethyl.

The α-hydroxy nitriles can, for instance, be synthesized by addition of hydrocyanic acid to ketones or aldehydes. A process for this is described, for instance, in US—A—2,745,865.

The conversion of the nitrile into the amide may take place immediately upon addition. It is not necessary, and often even undesirable, to isolate the cyanohydrin, as these compounds may be thermally unstable.

After the conversion into α-hydroxycarboxylic acid amides, which are stable at the usual temperatures, first the excess hydrogen peroxide can be decomposed using a reducing agent, following which the excess ammonia can simply be evaporated. Subsequently, if desired, separation of the optical isomers may take place. the L-α-hydroxy acid can be obtained by optical separation of the D,L-compound, for instance by application of an amidase-containing enzyme preparation obtained from a culture of *Rhodococcus erythropolis.* The optical separation of the D,L-compounds could, however, also be effected using physiochemical methods, in which the virtually pure racemic mixture can very simply be used as starting material.

The α-hydroxy-α-methylcarboxylic acid amides and corresponding acids, α-hydroxy-α-hydrogencarboxylic acid amides and corresponding acids and α-hydroxy-α-cycloalkylcarboxylic acid amides and corresponding acids can serve as intermediaries in the synthesis of products for the agricultural, the pharmaceutical, and the flavours and fragrances industries. By way of example of their applications mention may be made of D-2-hydroxy-3-(3,4-dihydroxyphenyl)-propionic acid in 'Rosmarinic acid' which can be used as antioxidant and in fighting inflammations.

The invention will be elucidated with reference to the following examples, without being restricted thereto.

## Example 1

An amount of 33 g of the hydroxy nitrile of benzaldehyde was added in about 1 hour to a solution of 25 ml concentrated ammonia and 60 ml of hydrogen peroxide (30%) in 250 ml water. The temperature of the reaction mixture was kept below 40°C by cooling. After 3 hours' stirring the hydrogen peroxide was reduced using 1 g palladium-on-carbon and the palladium-on-carbon was filtered off. The aqueous layer was subsequently evaporated at reduced pressure ($T \leq 40°C$) and a crystalline α-hydroxybenzylcarboxylic acid amide remained. After washing with 100 ml toluene the yield was 32.0 g (85%).

## Examples II—VI

As the α-hydroxy nitriles listed in Table 1 are thermally unstable, they preferably are used immediately after the synthesis, without further isolation. The addition of cyanic acid to an aldehyde or ketone using an acid catalyst is effected in an aqueous medium, with a yield of 50—80%, the α-hydroxynitrile being separable in an organic phase.

After the preparation of α-hydroxy nitriles, starting from aldehydes and ketones (Table 1), each α-hydroxycarboxylic acid amide was prepared in the same manner as in Example 1. The products obtained, and the yields, starting from the aldehyde or the ketone (the overall yield of both nitrile preparation and amide preparation according to the invention) are also shown in Table 1.

| Example | aldehyde or ketone | α-hydroxycarboxylic acid amide | | yield |
|---|---|---|---|---|
| | | $R_1$ | $R_2$ | |
| II | phenylpropionaldehyde | $C_6H_5CH_2CH_2$— | H— | 65% |
| III | isobutyraldehyde | $(CH_3)_2CH$— | H— | 60% |
| IV | acetone | —$CH_3$ | —$CH_3$ | 75% |
| V | cyclohexanone | —$CH_2$—$CH_2$—$CH_2$— | —$CH_2$—$CH_2$— | 70% |
| VI | methylethylketone | $CH_3$—$CH_2$— | —$CH_3$ | 50% |

## Claims

1. Process for the preparation of α-hydroxycarboxylic acid amides of the formula:

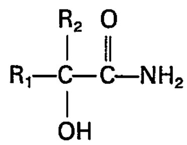

where $R_1$ represents alkyl, alkaryl, aryl, alkenyl, heteroalkyl or heteroaryl, whether or not substituted, $R_2$ represents hydrogen or methyl, or $R_1$ and $R_2$ together form cycloalkyl, by conversion of the corresponding cyanohydrins, characterized in that the conversion takes place in the presence of hydrogen peroxide and in the presence of 0.5—3 moles ammonia per mole of cyanohydrin.

2. Process according to claim 1, characterized in that it is carried out using 2—6 moles hydrogen peroxide per mole cyanohydrin.

3. Process according to claim 3, characterized in that use is made of about 2.2 moles hydrogen peroxide per mole cyanohydrin.

4. Process according to claims 1—3, characterized in that it is carried out using 0.5—2 moles ammonia per mole cyanohydrin.

5. Process according to any of claims 1—4, characterized in that it is carried out at a temperature between 0 and 50°C.

6. Process according to claim 5, characterized in that it is carried out at a temperature between 25 and 45°C.

## Patentansprüche

1. Verfahren zur Herstellung von α-Hydroxycarbonsäureamiden der Formel

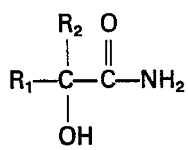

worin $R_1$ Alkyl, Alkaryl, Aryl, Alkenyl, Heteroalkyl oder Heteroaryl, egal ob substituiert oder nicht, bedeutet, $R_2$ Wasserstoff oder Methyl darstellt oder $R_1$ und $R_2$ miteinander Cycloalkyl bilden, durch Reaktion der entsprechenden Cyanhydrine, dadurch gekennzeichnet, daß die Reaktion in Anwesenheit von Wasserstoffperoxid und in Anwesenheit von 0,5 bis 3 Molen Ammoniak pro Mol Cyanhydrin durchgeführt wird.

2. Verfahren nach Anspruch 1, dadurch gekennzeichnet, daß es unter Verwendung von 2 bis 6 Molen Wasserstoffperoxid pro Mol Cyanhydrin durchgeführt wird.

3. Verfahren nach Anspruch 2, dadurch gekennzeichnet, daß etwa 2,2 Mole Wasserstoffperoxid pro Mol Cyanhydrid verwendet werden.

4. Verfahren nach Ansprüchen 1 bis 2, dadurch gekennzeichnet, daß es unter Verwendung von 0,5 bis 2 Molen Ammoniak pro Mol Cyanhydrin durchgeführt wird.

5. Verfahren nach einem der Ansprüche 1 bis 4, dadurch gekennzeichnet, daß es bei einer Temperatur von 0 bis 50°C durchgeführt wird.

6. Verfahren nach Anspruch 5, dadurch gekennzeichnet, daß es bei einer Temperatur von 25 bis 45°C durchgeführt wird.

**Revendications**

1. Procédé pour la préparation d'amides d'acides α-hydroxycarboxyliques de la formule:

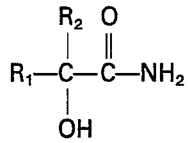

dans laquelle $R_1$ représente un alkyle, un alkaryle, un aryle, un alcényle, un hétéroalkyle ou un hétéro-aryle, qu'il soit substitué ou non, $R_2$ représente de l'hydrogène ou du méthyle, ou bien $R_1$ et $R_2$ forment conjointement un cycloalkyle, par conversion des cyanhydrines correspondantes.

2. Procédé selon la revendication 1, caractérisé en ce qu'il est mis en oeuvre en utilisant de 2 à 6 moles de peroxyde d'hydrogène par mole de cyanhydrine.

3. Procédé selon la revendication 2, caractérisé en ce que l'on utilise environ 2,2 moles de peroxyde d'hydrogène par mole de cyanhydrine.

4. Procédé selon les revendications 1 à 3, caractérisé en ce qu'il est mis en oeuvre en utilisant 0,5 à 2 moles d'ammoniac par mole de cyanhdrine.

5. Procédé selon l'une quelconque des revendications 1 à 4, caractérisé en ce qu'il est mis en oeuvre à une température entre 0 et 50°C.

6. Procédé selon la revendication 5, caractérisé en ce qu'il est mis en oeuvre à une température entre 25 et 45°C.